# EUROPEAN PATENT APPLICATION

(11) **EP 2 520 312 A1**
(43) Date of publication of application: **07.11.2012**
(21) Application number: 12178534.9
(22) Date of filing: 20.02.2007
(51) Int. Cl.: A61K 47/00, A23L 1/22, A23G 4/20, A23L 1/00, A23G 4/06, A23G 3/54, A61K 9/00

(54) **A process for manufacturing a delivery system for active components as part of an edible composition**

(30) Priority: 28.06.2006 US 816873 P
(62) Divisional of application: 07757218.8
(71) Applicant: Kraft Foods Global Brands LLC, Northfield, IL 60093 (US)
(72) Inventor: BOGHANI, Navroz, Flanders, NJ New Jersey 07836 (US); BUNKERS, Joseph, M., Caledonia, IL Illinois 61011 (US); DUGGAN, James A., Machesney Park, IL Illinois 61115 (US); BARRERA, George, A., Caledonia, IL Illinois 61011 (US); JANI, Bharet, East Brunswick, NJ New Jersey 08816 (US); GEBRESELASSIE, Petros, Highland Park, NJ New Jersey 08904 (US)
(74) Representative: Wilson Gunn

(57) **Abstract**

A method of manufacturing an edible delivery system of at least one active component encapsulated by extrusion in an encapsulating material, which is useful, for example, for providing delayed and/or controlled release of the active is described.
The corresponding delivery system and the corresponding edible composition, such as a chewing gum, are also described.

## Description

### BACKGROUND

### FIELD

A method of manufacturing a delivery system of at least one active component encapsulated in an encapsulating material, which is useful, for example, for providing delayed and/or controlled release of the active is described.

### DESCRIPTION OF THE BACKGROUND

High intensity sweeteners generally have a sweetening intensity greater than sugar (sucrose) and a caloric value lower than that of sugar at equivalent sweetness levels. In some situations, it is especially desirable to control the release of high intensity sweeteners in compositions since the high sweetness levels can easily overwhelm the consumer. Moreover, the controlled release of the sweetener provides desirable masking of unpleasant tasting materials and may help bring out flavor characteristics of other ingredients. Because each high intensity sweetener is chemically and physically distinct, each is a challenge to use in an edible composition and each exhibits one or more shortcomings, which may be moderated by encapsulation.

For example, many high intensity sweeteners lose their sweetness intensity rapidly when used in edible compositions such as chewing gums and confections with certain flavors. Encapsulation can modulate and prolong release to provide a more desirable taste profile. Some high intensity sweeteners such as saccharin, stevioside, acesulfame-K, glycyrrhizin, and thaumatin have an associated bitter taste or off-note. Certain high intensity sweeteners are also unstable in the presence of certain chemicals including aldehydes and ketones, and sensitive to exposure to environmental conditions including moisture. Solid sucralose is known to turn dark during prolong storage upon exposure to heat and ambient air. Encapsulation can be used to isolate unstable compounds to prevent degradation and prolong shelf life.

Typically, the taste profile of a high intensity sweetener can be described as a rapid burst of sweetness. Usually, high intensity sweeteners reach their peak sweet taste rapidly, with the intensity of sweet taste rapidly declining soon thereafter. The initial rapid burst can be unpleasant to many consumers as the strong sweet taste tends to overpower the other flavors that may be present in the edible composition. The relatively rapid loss of sweetness can also result in a bitter aftertaste. For this reason, it may be desirable to encapsulate high intensity sweeteners with an encapsulating material in order to modulate and prolong the release rate and to chemically stabilize and enhance the overall taste profile.

### SUMMARY

The present invention is a significant advance in the art by providing an improved method for preparing a delivery system that provides controlled and/or delayed release of one or more active agents. Non-limiting examples of the active agent are sweeteners including high intensity sweeteners, acids, flavorants, pharmaceuticals, therapeutic agents, vitamins, minerals, a tooth whitener or cleaner, breath fresheners, cooling agents, warming agent, a sensate and others.

The present invention provides a new approach to the manufacture of a delivery system that can be used to control and/or release of an active component in edible compositions such as, for example, chewing gum, confectionery compositions, and other edible products. The active component(s) and materials used to partially or completely encapsulate the same provide a delivery system(s) that enables exceptional control of the release of the active component over a wide range of delivery systems and takes into account the use of a range of encapsulating materials and additives that may be used to formulate the delivery system. The encapsulated active components are preserved until release is desirable and therefore can be protected against moisture, reactive compounds, pH changes and the like. When the active component is a sweetener, the delivery system can be tailored to provide consistent sustained release, thus extending the time the sweetener is released or available to provide an edible composition which provides a long lasting desirable taste profile, increased salivation and/or overall enjoyment of the taste.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:

Figure 1 is a diagrammatic elevation view showing one embodiment of a method and apparatus for manufacturing a delivery system.

Figure 2 is a diagrammatic elevation view showing another embodiment of a method and apparatus, including optional components, for manufacturing a delivery.

Figure 3 is a diagrammatic elevation view showing another embodiment of a method and apparatus for manufacturing a delivery.

Figure 4 is a diagrammatic elevation view showing another embodiment of a method and apparatus for manufacturing a delivery system.

Figure 5 is a diagrammatic elevation view showing another embodiment of a method and apparatus for manufacturing a delivery system.

Figure 6 is a diagrammatic elevation view showing another embodiment of a method and apparatus for manufacturing a delivery system.

### DETAILED DESCRIPTION

As used herein, it is understood that the terms "comprising" and "comprises" is an open transitional phrase permitting the inclusion of other elements not specifically recited and thus within the scope of the embodiment and/or claim.

U.S. application serial no. 10/719,298 filed November 21, 2003 and PCT/US20041037185 filed November 22, 2004 are incorporated herein by reference.

Referring now to the drawings, wherein like reference numerals designate identical or corresponding parts throughout the several views.

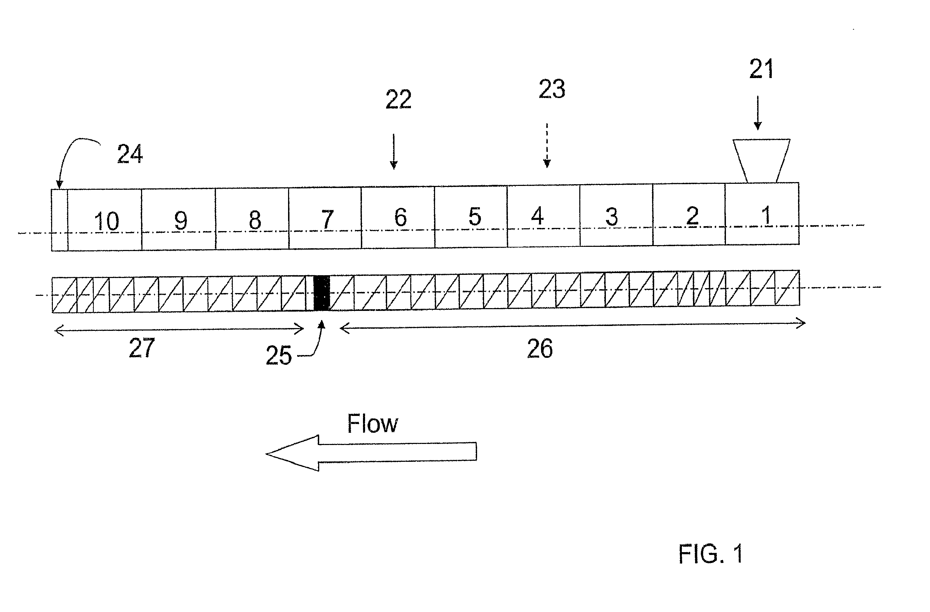

Referring to Figure 1, a representative continuous mixer is shown having ten mixing zones (1-10), each zone representing a different feed inlet for the components of the delivery system, different conveying and/or mixing elements within the mixer or a combination of these. The number of zones can vary depending on the particular encapsulating material, active component(s) and/or other components added to the continuous mixer. The number of zones can be adjusted to reduce or increase the residence time in the mixer to, for example, increase or decrease the temperature within the mixer, and/or increase or decrease the amount of mixing within the mixer. In this Figure, encapsulating material **21** is added to the mixer through a feed inlet or hopper in mixing zone 1 where it is conveyed through a conveying region **26** where towards the end of the conveying region **26** the active component **22** is fed into the mixer. The encapsulating material and the active component are blended in a mixing region **25** and then conveyed through another conveying region **27** to the end plate of the mixer **24**. In some embodiments, mixing also may occur in the conveying region **26** and/or the conveying region **27**. While shown in zone 7 within the continuous mixer, the position of the mixing region **25** can be moved to different zones within the mixing region and preferably is positioned in the continuous mixer downstream of the addition of at least some of the encapsulating material, the active component, and other ingredients (if used). Within the mixing region **25**, one or more mixing elements are included. Non-limiting examples of such mixing elements include Kneading, (neutral or pitched RH or LH with single, double, triple, quad or poly lobe designs, shoulder, ftx and multipurpose configurations), combinations like segment elements, blade or cut that give turbine mixing qualities (for example, zme, tme, zb, zs, sme, sfe, distributive mixers like igel), twin-screw, single screw, blade-and-pin, barrier and others as known in the art. The length of conveying region **26** can be adjusted to optimize the melting of or maintaining of temperature of the encapsulating material. In addition, the length of conveying region **27** can be adjusted to optimize, increase or decrease the amount of mixing of the encapsulating material and active component (and other ingredients if added) for purposes of reducing or increasing temperature, increasing or decreasing encapsulation, etc. In some embodiments, the conveying region **27** may not be used. Also shown is the optional feeding of one or more additional liquid and/or solid ingredients **23** that may be incorporated into the delivery system as described herein.

The continuous mixer useful in the process of the present invention can be a twin-screw extruder, single-screw extruder, blade-and-pin and other types of mixers that can provide continuous throughput. For example, such mixers can be configured, in one embodiment, to have conveying regions **26** and **27** at the start and end of the mixer with the appropriate mixing element, e.g., twin-screw, single-screw or blade-and-pin, as the mixing region **25**. Other configurations and mixers are also possible. For example, an in-line mixer can be used in which the ingredients are fed into the mixer and create a distributive and shear mixing. Such in-line mixer may be linked with a continuous mixer to produce our product. In other embodiments, two or more continuous mixers or at least one continuous mixer coupled with a batch mixer can be used to prepare the delivery systems as described herein.

In another embodiment, the continuous mixer can include a restrictive element within zone 1 and/or in a downstream position relative to mixing zone 1 to increase back pressure within the mixer, increase and/or optimize the volume of the material in the continuous mixer, e.g., in certain regions where needed. For example, a mixing restriction element may be located in conveying region **27**. Such restrictive elements are known in the art and may include elements with reverse flight (relative to the direction of flow in the mixer) and depending on the length or degree of pitch will increase the reverse flow and back pressure. In one embodiment, the continuous mixer can contain solely conveyance elements, e.g., devoid of a mixing region such as a twin-screw element, coupled with one or more restrictive elements. In this embodiment, the at least one restrictive element is located at a position in the mixer downstream of the point where the components of the delivery system are fed into the continuous mixing apparatus.

When configuring the continuous mixer to manufacture the delivery system, the encapsulating material can be added to the mixer in melted form, can be added in solid form and melted in the mixer, or a combination of these. If added in melted form, which can reduce the shear of the material in the continuous mixer, the encapsulating material (e.g., polyvinyl acetate and/or others) can be introduced into the mixer through a specially adapted side-feeder. The arrangement of conveying regions, including number, mixing regions (if included), are preferably optimized to gently melt the encapsulating material to a temperature to facilitate workability and mixing of the encapsulating material with the active component before at least some of the active component is added. As one of skill in the art will recognize the temperature should not be too high and/or maintained at a high temperature whereby the active component would substantially decompose before exiting the mixer. Thus, the temperature of the material in the mixer is preferably maintained to minimize the degradation of the active component. In certain embodiments the temperature of the material in the mixer (or the mixer itself depending on the point of measurement) is maintained at a temperature such that the amount of active component is at least 90% by weight relative to the amount of active component added initially to the mixer. In further embodiments, the temperature is maintained such that the amount of active component is at least about 50%, including 55, 60, 65, 70, 75, 80, and 85% by weight, as well as all values and ranges there between, relative to the amount of active component added initially to the mixer.

In certain embodiments, one or more lubricants are added to the continuous mixer to reduce and/or avoid the material introduced into the mixer from sticking on the inside surface of the mixer. As lubricants, various materials can be added and include mono and di Glycerides, fats (with or without hydrogenation), waxes, triacetin, lecithin, aceticacid esters of monodiglycerides, lactic acid esters of monodiglycerides, citric acid esters of monodiglycerides, diacetyl tartaric acid esters of monodiglycerides, succinic acid esters of monodiglycerides, salts of fatty acids (Na, K, Ca, Mg), polyglycerol esters of fattyacids (e.g. triglycerol monostearate), propylene glycolesters of fatty acids, sorbitan monostearate, sorbitan tristearate, Polysorbates. The lubricants can be added anywhere in the extruder and in certain embodiments, in the middle of extruder and/or after the encapsulating material has been added. The amount of lubricant can vary depending on the types of encapsulating material, active and/or length of mixer/mixing process. In certain embodiments, the lubricant is employed in an amount of 0.5 - 30% by weight of the material be added to the mixer, preferably, the amount ranges from about 2 to about 5%. The amount of the lubricant should preferably not exceed a level such that the delivery system is weakened.

The ingredients, including encapsulating material, active component and/or additional ingredients (if used) may be fed into the continuous mixer using commonly used devices for this purpose. For example, pumps, gravity feeders, side feeders and extruders may be used. In one embodiment, the active component and/or other ingredients are fed into the continuous mixer using a side feeder. Side feeders used to feed ingredients into a mixer are known in the field, including, for example, extruders, single or twin screw, or mixers composed of one or more conveying elements. Rework or recycled delivery system material can be added to the continuous mixer with the encapsulating material **21**, e.g., in mixing zone 1 and/or can be added to the continuous mixer at least prior to the addition of the active component and/or additional ingredients such as fat, if used.

In certain embodiments, the continuous mixer may include one or more sensors for sensing, detecting, and/or measuring one or more properties of the mixer, the material added to the mixer, and/or the material being processed through the mixer, including for example, the rate at which the components are fed into the mixer, the pressure within the mixer, flow rate of material in the mixer, temperature of the mixer and/or the material in the mixer, as well as other processing parameters. These sensors can be used to control one or more of these parameters by providing information to the operator, such as a light or alarm, and/or the information processed by the sensors can be fed back to a control module (or the sensor can be configured to do this without a separate control) where the appropriate parameter is adjusted to achieve the desired objective. In certain instances, one or more of the parameters being sensed can be controlled according to preset or preprogrammed configuration(s).

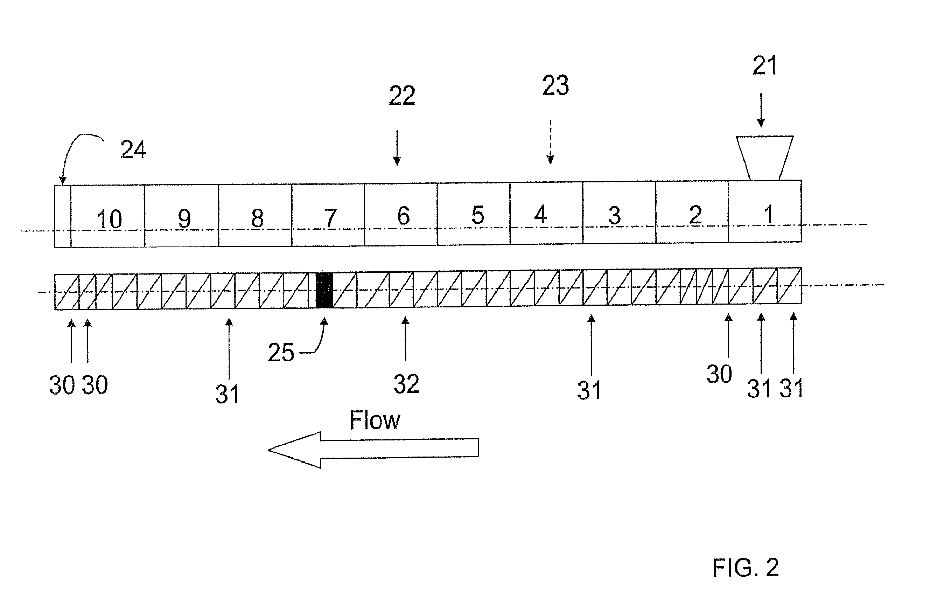

Referring to Figure 2, various positions of conveying elements within the conveying regions are shown and include narrow pitch conveying elements **30**, medium pitch conveying elements **31**, and wide pitch conveying elements **32**. The pitch of the conveying elements are not particularly restricted to certain specific sizes provided that in relation to each other there are some conveying elements with more or less pitch accordingly. Thus, a narrow pitch conveying element has a pitch less than a high and medium pitch conveying element, a medium pitch element has a pitch between a high and narrow pitch conveying element, and a high pitch conveying element has a pitch greater than a narrow and medium pitch conveying element. For example, a narrow pitch element could have a pitch of 30 mm long with an overall length of 30 mm; a high pitch conveying element could have a pitch of 135 mm with an overall length of 67.5 mm; and a medium pitch conveying element would have a pitch in between these two, e.g., a pitch of 90 mm and a length of 90 mm.

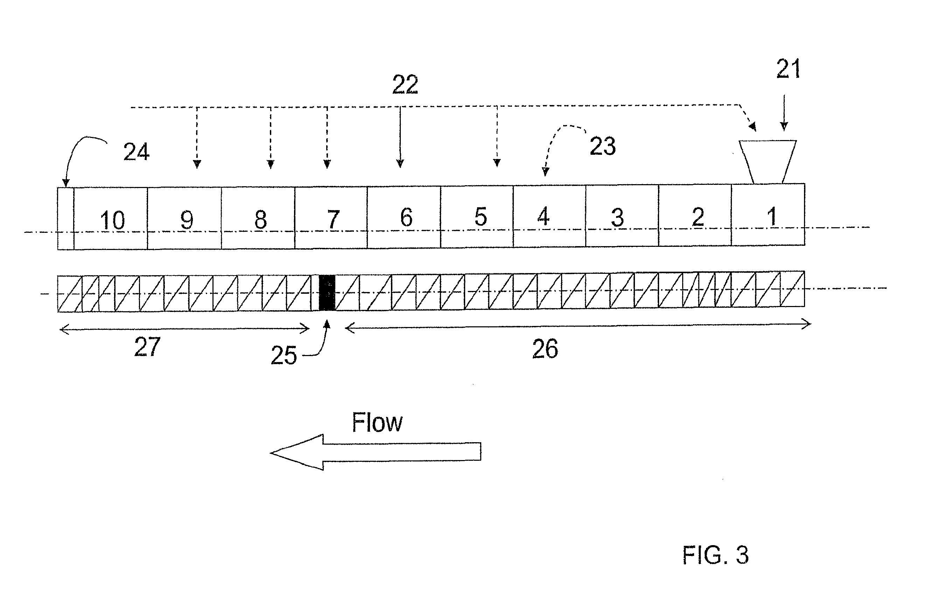

Referring to Figure 3, the optional feeding of the active component **22** through two or more feed ports in the continuous mixer are shown. For example, using sweeteners as an example of the active component, it may be desirable to incorporate sweeteners into the encapsulating material at different stages to provide multiple levels of sweetener release. In this embodiment, adding the sweetener to the mixer at an early point in the process can result in a more delayed release relative to the sweetener added to the mixer at a later stage in the process.

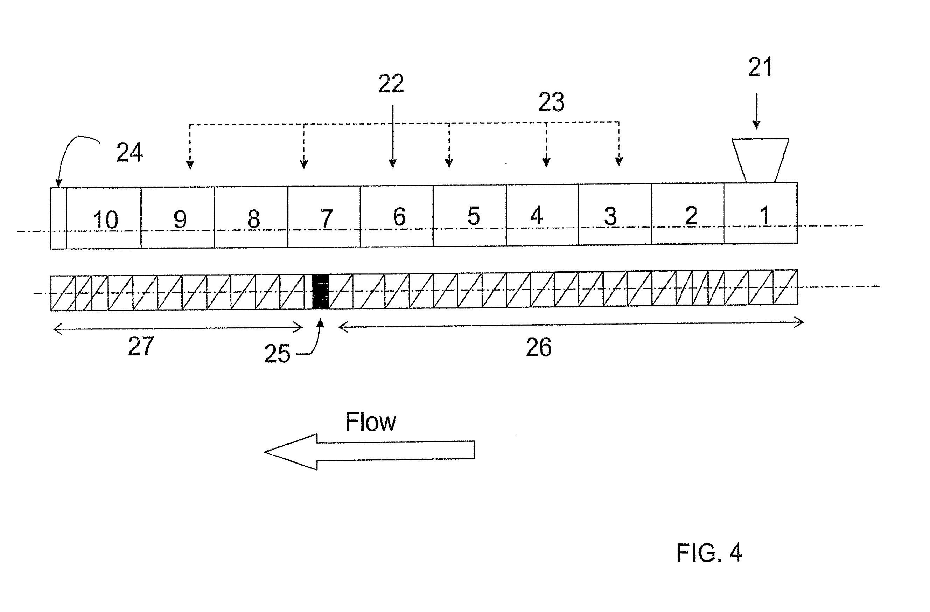

Referring to Figure 4, the optional feeding into several regions of the continuous mixer of one or more additional liquid and/or solid ingredients **23** that may be incorporated into the delivery system is shown. Examples of liquid and/or solid ingredients **23** that may be incorporated include, in some embodiments, fats, oils, waxes, tensile strength modifying agents and other ingredients as described herein, for example, glycerol monostearate. For example, as described herein, the addition of fats and/or oils to the mixer during processing of the encapsulating material can modify the tensile strength of the delivery system produced to yield a desired release rate.

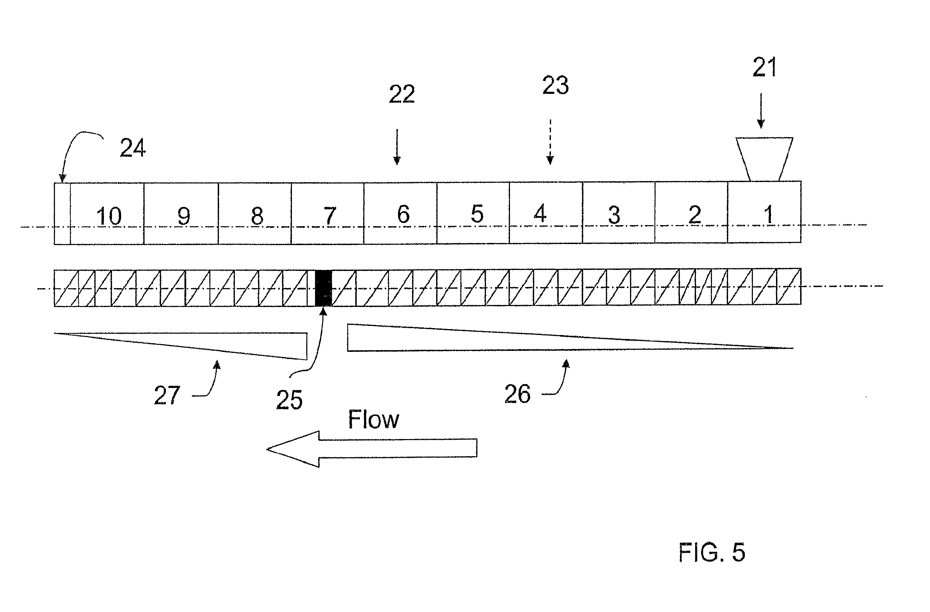

Referring to Figure 5, relative pitch of the conveying elements in conveying regions **26** and **27.** In conveying region **26**, the conveying elements begin as narrow pitch elements and gradually increase to wide pitch elements up to the mixing element **25**. In conveying region **27**, the conveying elements, after the mixing element **25**, begin as wide pitch elements gradually decreasing to narrow pitch elements towards the end of the mixer. Depending on the configuration of the mixer, the conveying elements in the conveying regions **26** and **27**, and the addition of ingredients, mixing may occur in the conveying region **26** and/or the conveying region **27**.

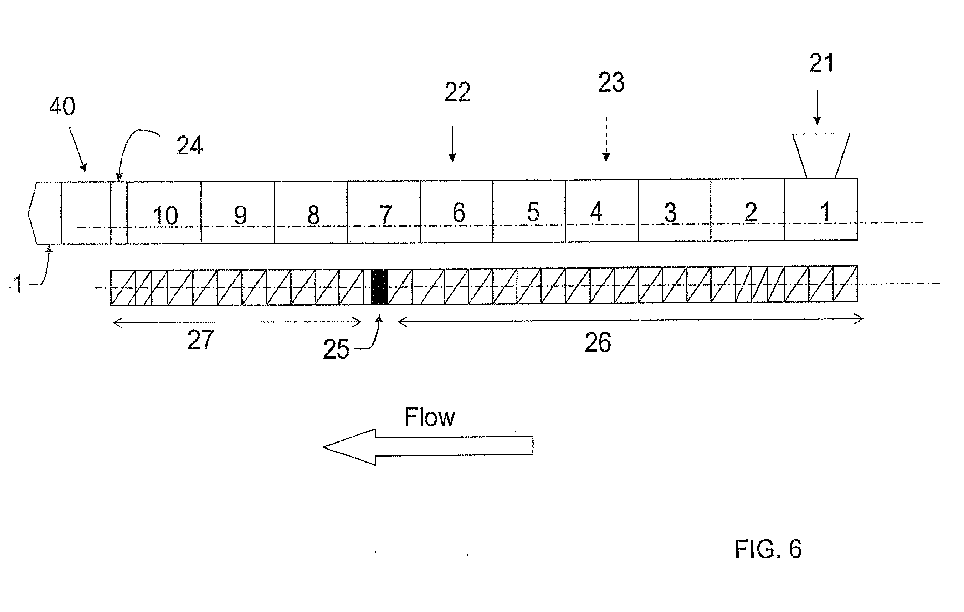

Referring to Figure 6, a pump **40** is shown positioned at the end of the mixer to convey the material from the mixer to downstream processing. The pump may be a low shear pump and can act to increase uniformity of the material through a die **41** and can also increase cooling efficiency of the material passing from the mixer by reducing pressure build up at the point of discharge from the mixer. Furthermore, the pump **40** can facilitate control of the discharge pressure from the mixer, act as a control for flow of material through the mixer, and mixing in the continuous mixer when incorporated with the speed of operation of the mixer. In one embodiment, the pump speed can be controlled based on the speed and/or pressure in the continuous mixer. This control can be separate or in conjunction with other process parameters as described herein.

In some embodiments, the output from the mixer may be cooled and ground for use as an ingredient in an edible composition, such as chewing gum. In further embodiments, the ground up material may be coated with a powder to minimize clumping and/or to act as a further processing aid.

In some embodiments, the method of manufacturing a delivery system includes adding the encapsulating material and the at least one active component to a continuous mixer; mixing the encapsulating material with the at least one active component such that the at least one active component is at least partially encapsulated by the encapsulating material.

In some embodiments, there is provided a method of manufacturing a delivery system for inclusion in an edible composition such as a chewing gum composition or confectionery composition having at least one active component at least partially encapsulated by an encapsulating material.

In some embodiments, the method for manufacturing the delivery system includes feeding the encapsulating material and the at least one active component into a continuous mixer; wherein the continuous mixer comprises at least a first and a second conveying region and a mixing region between the at least a first and second conveying regions, wherein the first conveying region is upstream from the second conveying region in the continuous mixer, wherein at least a portion of the encapsulating material is fed into a first conveying region and at least a portion of the at least one active component is fed at or upstream of the mixing region but downstream of where the encapsulating material is fed into the continuous mixer, mixing the encapsulating material and at least one active component in the mixing region; and conveying the encapsulating material and the at least one active component through the second conveying region thereby producing a delivery system.

There is also provided a method of manufacturing an edible composition, for example, a confectionary or chewing gum composition by mixing the delivery system manufactured according to the description provided herein with at least one edible composition forming component, e.g., a gum base, to produce an edible composition.

Although one embodiment relates to chewing gum compositions, confectionery compositions and beverages, the methods and apparatus disclosed herein can be utilized to produce a variety of edible compositions including, but not limited to, food products, foodstuffs, nutrient-containing compositions, pharmaceuticals, nutraceuticals, vitamins and other products that may be prepared for consumption by the consumer. As used herein, chewing gum compositions include bubble gum compositions. Because the delivery system may be readily incorporated into an edible composition, the edible compositions which may benefit from and are encompassed by the present invention are wide ranging as indicated above.

The term "delivery system" as used herein is meant to encompass the encapsulating material and at least one active component encapsulated therein as well as other optional additives used to form the delivery system as hereinafter described. It will be understood that the edible compositions of the present invention may contain a plurality of delivery systems with each delivery system containing a single or multiple active components.

The term "encapsulating material" is meant to encompass any one or more edible water insoluble materials capable of forming a solid coating or film as a protective barrier around the active component.

The present invention is directed generally to the manufacture of a delivery system as defined herein for use in edible compositions, which comprises an encapsulating material and an active component encapsulated by the encapsulating material. The delivery system is formulated to provide consistent controlled release of the active component over a preselected period of time, such as an extended period of time. This period of time may vary depending on the type of product in which the delivery system is incorporated, the type of encapsulating material, the type of active, other ingredients (e.g., fats) in the product, etc. One of skill in the art, based on the disclosure herein can adjust the delivery system and mixer configuration to achieve the desired effect.

In some embodiments, an extended period of time as used herein, relates to an increased release of the active ingredient from the delivery system for over a greater period of time than previously described systems and can be at least 15 minutes, including at least 20 minutes, at least 25 minutes, at least 30 minutes, as well as all values and ranges there between, for example, about 25 to 30 minutes, 45 to 60 minutes, or more. Furthermore, the delivery system of the present invention also provides a way to not only deliver active agents over a prolonged period of time but also maintain an increased intensity of the active ingredient over the extended period of time. For example, if the active ingredient is a flavor or sweetener. In one aspect of the invention, the amount of active agent released can vary during the extended period of time. For example, at an early stage of delivery the amount of active component released (based on the total amount present in the delivery system at that time) can be greater than the amount of active component released during subsequent or later periods (based on the total amount present in the delivery system at that time).

In one embodiment, the extended period of time results in retaining at least about 5% of the at least one active component after 30 minutes from the start of delivering the active component in the edible composition, such as the start of chewing a chewing gum composition, including at least about 10 %, 15%, 20 %, 25 %, 30 %, or more after 30 minutes. In another embodiment, the extended period of time results in retaining at least about 10% of the at least one active component after 20 minutes from the start of delivering the active component, including at least about 15%, 20 %, 25 %, 30 %, 40%, 50 % or more after 20 minutes. In another embodiment, the extended period of time results in retaining at least about 30% of the at least one active component after 15 minutes from the start of delivering the active component, including at least about 30 %, 40%, 50 %, 60 %, 70%, 75% or more after 15 minutes.

In another embodiment, using sweetener in chewing gum as an example, the extended period of time results in a perceived sweetness intensity during at least the entire period of time noted above, e.g., at least about 15 minutes, at least about 20 minutes, at least about 30 minutes, etcetera from the start of chewing the chewing gum composition. Moreover, extending the period of time that the sweetener is available during chewing may extend the amount of time that flavor is perceived by the consumer.

The manufactured delivery system facilitates the controlled release of the active component in a wide variety of edible compositions including chewing gum compositions, food products, confectionery compositions, pharmaceutical compositions, beverages, foodstuffs, nutrient-containing compositions, vitamins, nutraceuticals and the like.

The delivery system developed in accordance with the present invention may be selected, depending in part on the active component and the release rate of the active component desired, from a standard of known delivery systems containing the active component at known release rates. The active components which may be incorporated as part of the delivery system may be selected from sweeteners including high intensity sweeteners, acids, flavorants, pharmaceuticals, therapeutic agents, vitamins, minerals, a tooth whitener or cleaner, breath fresheners, cooling agents, warming agent, a sensate and other materials that would benefit by coating for protection, controlled release and/or for taste masking. The active components include nicotine useful for the treatment of addiction to tobacco products and caffeine typically found in coffee and/or beverages. In one embodiment of the present invention, the active component is a sweetener, for example a high intensity sweetener such as neotame, aspartame, sucralose, acesulfame potassium and others as described herein.

The delivery system for delivering an active component can be formulated to ensure an effective sustained release of the active component based on the type and amount of the active component and desired release rate. For example, it may be desirable to affect the controlled release of a high intensity sweetener over a period of 25 to 30 minutes to ensure against a rapid burst of sweetness which may be offensive to some consumers. A shorter controlled release time may be desirable for other type of active components such as pharmaceuticals or therapeutic agents, which may be incorporated into the same edible composition by using separate delivery systems for each active component. In accordance with the present invention, delivery systems may be formulated based on a range of release rates relative to a standard. The standard may comprise a series of known delivery systems having, for example, a polymer encapsulating material having specific hydrophobicity and/or tensile strengths over a range. Each of the delivery systems of the standard will be associated with a particular release rate or ranges of release rates.

In one embodiment, the manufacture of edible compositions includes incorporating a plurality of delivery systems to deliver a plurality of separate active components including active components which may be desirably released at distinctly different release rates. The active components can be the same or different. Different delivery systems may use different active components and/or different encapsulating materials.

For example, high intensity sweeteners may desirably be released over an extended period of time (e.g., 20 to 30 minutes) while some pharmaceuticals are desirably released over a significantly shorter period of time.

In certain embodiments of the present invention, the delivery system can be manufactured such that the release of the at least one active agent is at specific rates relative to the time of delivery. For example, in one embodiment, the delivery system can be prepared such that the release of the at least one active agent is released at a rate of 80% over the course of 15 minutes, 90% over the course of 20 minutes, and/or a 95% over the course of 30 minutes. In another embodiment, the delivery system can be prepared such that the one or more active agents are released at a rate of 25 % over the course of 15 minutes, 50% over the course of 20 minutes and/or 75% over the course of 30 minutes. For example, using chewing gum as an example, the same sweetener can be incorporated into two different delivery systems, one of which provides an early release and second providing a more delayed release to contribute to longer lasting perceived sweetness and/or flavor by the consumer.

Polymers which may be used in as the encapsulating material include, but are not limited to polyvinyl acetate, polyethylene, crosslinked polyvinyl pyrrolidone, polymethylmethacrylate, polylactidacid, polyhydroxyalkanoates, ethylcellulose, polyvinyl acetatephthalate, polyethylene glycol esters, methacrylicacid-co-methylmethacrylate, and the like; homo- and co-polymers of, for example, vinyl acetate, vinyl alcohol, ethylene, acrylic acid, methacrylate, methacrylic acid; vinyl acetate/vinyl alcohol copolymer, ethylene/vinyl alcohol copolymer, ethylene/acrylic acid copolymer, ethylene/methacrylate copolymer, ethylene/methacrylic acid copolymer; ethylene vinyl acetate, and combinations of these.

In some embodiments, the encapsulating material may be present in amounts of from about 0.2% to 10% by weight based on the total weight of the edible composition, including 0.3, 0.5, 0.7, 0.9, 1.0, 1.25, 1.4, 1.7, 1.9, 2.2, 2.4.5, 2.75, 3.0, 3.5, 4.0, 4.25, 4.8, 5.0, 5.5, 6.0, 6.5, 7.0, 7.25, 7.75, 8.0, 8.3, 8.7, 9.0, 9.25, 9.5, 9.8 and all values and ranges there between, for example, from 1% to 5% by weight. The amount of the encapsulating material will, of course, depend in part on the amount of the active component which must be encapsulated and/or the degree of encapsulation desired. The amount of the encapsulating material with respect to the weight of the delivery system, is from about 30% to 99%, including 35, 40, 45, 50, 55, 60, 65, 70, 75, 80, 85, 95, 97 and all values and ranges there between, for example, from about 60% to 90% by weight.

The active component can be entirely encapsulated within the encapsulating material or incompletely encapsulated within the encapsulating material provided the resulting tensile strength of the delivery system meets the criteria set forth hereinabove. The incomplete encapsulation can be accomplished by modifying and/or adjusting the manufacturing and mixing process to get partial coverage of the active component. In some embodiments, different mixing and/or conveying elements may impact the amount of encapsulation. In addition, a longer residence time in the mixer may impact the degree of encapsulation of the active components. In certain embodiments, the active agent (e.g., sweeteners such as sucralose) can be premixed with one or more processing aids to prevent agglomeration of the material when added. Additionally or alternatively, the active agent can be fed into the mixer along with the one or more processing aids. Additionally or alternatively, the processing aid can be added to the inner walls of the device used to feed the active into the mixer. Non-limiting examples of suitable processing aids include Talc, Dicalciumphosphate, high melting point sugar alcohols, such as Mannitol, microparticle size corn starch, microcrystalline cellulose, confectioners sugar. The amount of processing aid can vary depending on the agglomeration properties of the active, useful levels of the processing aid range from about 0.5 to about 50% by weight of the active being added, including from about 1 to about 10%, including 2, 3, 4, 5, 6, 7, 8, and 9%.

In one aspect of the present invention, the release of the active component can be controlled by formulating the delivery system based on the hydrophobicity of the encapsulating material, e.g., polymer being added to the mixer. By adding highly hydrophobic polymers, the release times of the active component can be increased. In a similar manner, using encapsulating material that is less hydrophobic, the active component can be released more rapidly. Hydrophobicity can be quantitated by the relative water-absorption measured according to ASTM D570-98. Thus, by selecting encapsulating material with relatively higher water-absorption properties and adding that to the mixer, the release of the active component contained in the produced delivery system can be delayed compared to those encapsulating materials having lower water-absorption properties. In certain embodiments, to delay the release of the at least one active component from the delivery system a water absorption of from about 50 to 100% (as measured according to ASTM D570-98). Moreover, to increase the relative delivery rate, the encapsulating material can be selected such that the water absorption would be from about 15 to about 50 % (as measured according to ASTM D570-98). Still further, in other embodiments, the water absorption properties of the encapsulating material can be selected to be from 0.0 to about 5% or up to about 15% (as measured according to ASTM D570-98). In other embodiments, mixtures of two or more delivery systems formulated with encapsulating material having different water-absorption properties can also be used in subsequent incorporation into an edible composition.

For example, if ethylene-vinyl acetate is the encapsulating material, the degree of hydrophobicity can be controlled by adjusting the ratio of ethylene and vinyl acetate in the copolymer. The higher the ethylene:vinylacetate ratio, the slower the release of the active component. Using vinylacetate/ethylene copolymer as an example, the ratio of the vinylacetate/ethylene in the copolymer can be from about 1 to about 60 %, including ratios of 2.5, 5, 7.5, 9, 12, 18, 23, 25, 28, 30, 35, 42, 47, 52, 55, 58.5 % and all values and ranges there between.

In a further embodiment, the manufacture of a delivery system can be prepared based on the manipulation and selection of the tensile strength of the encapsulating material to provide a delayed and/or controlled release of the active component, which can be combined with or independent of the hydrophobic character discussed hereinabove. Thus, the controlled and/or delayed release of the active component can be controlled by selecting a predetermined tensile strength and a predetermined hydrophobicity of the encapsulating material.

As used herein, the term "tensile strength" means the maximum stress a material subjected to a stretching load can withstand without tearing. A standard method for measuring tensile strength of a given substance is defined by the American Society of Testing Materials in method number ASTM-D638.

The predetermined tensile strength is determined based, in part, on the active component and the desired release time of the same. The predetermined tensile strength may be selected from a standard comprised of one or more delivery systems with each standard delivery system having a known release rate of the desired active component. The delivery system further provides the active component with a protective barrier against moisture and other conditions such as pH changes, reactive compounds and the like, the presence of which can undesirably degrade the active component.

It will be understood that a plurality of delivery systems may be prepared in this manner each containing a different active component by utilizing a comparison with standard delivery systems containing such different active components.

By maintaining the tensile strength of the delivery system within a preselected desirable range, the active component can be released from the composition in a highly controlled and consistent manner. By focusing on the tensile strength of the delivery system, the process for selecting and formulating suitable delivery systems is enhanced in a manner which effectively reduces the need for trial and error experimentation typically necessary in prior art systems.

The desired tensile strength of the delivery system can be readily determined within a desired range. In one embodiment of the present invention, the tensile strength of the delivery system is at least 6,500 psi, including 7500, 10,000, 20,000, 30,000, 40,000, 50,000, 60,000, 70,000, 80,000, 90,000, 100,000, 125,000, 135,000, 150,000, 165,000, 175,000, 180,000, 195,000, 200,000 or more and all ranges and subranges there between, for example a tensile strength range of 6,500 to 200,000 psi. The formulation of a delivery system with a desirable tensile strength can be made from a variety of encapsulating materials and at least one additive which hereinafter are referred to as "at least one tensile strength modifying agent or modifier," Which may be added to the mixer with the encapsulating material and/or active, preblended with the encapsulating material prior to feeding into the mixer and/or active, and/or added separately to the mixer as the additional ingredient 23 noted in the Figures described herein. The at least one additive may be used to formulate the delivery system by modifying the tensile strength of the delivery system, including tensile strength-lowering materials such as fats, emulsifiers, plasticizers (softeners), waxes, low molecular weight polymers, and the like, in addition to tensile strength increasing materials such as high molecular weight polymers. In addition, the tensile strength of the delivery system can also be fine tuned by combining different tensile strength modifiers to form the delivery system. For example, the tensile strength of high molecular weight polymers such as polyvinyl acetate may be reduced when tensile strength lowering agents such as fats and/or oils are added to the delivery system.

In one embodiment, at least one tensile strength modifying agent is added to the mixer in an amount sufficient such that the release of the one or more active agents contained in the delivery system produced from the process is released at a rate of 80% over the course of 15 minutes, 90% over the course of 20 minutes, and/or a 95% over the course of 30 minutes. In another embodiment, the at least one tensile strength modifying agent is added to the mixer in an amount sufficient such that the one or more active agents are released at a rate of 25 % over the course of 15 minutes, 50% over the course of 20 minutes and/or 75% over the course of 30 minutes.

In another embodiment of the present invention, the at least one tensile strength modifying agent is present in the delivery system in an amount sufficient such that the tensile strength of the delivery system is at least about 6,500 psi, including 7500, 10,000, 20,000, 30,000, 40,000, 50,000, 60,000, 70,000, 80,000, 90,000, 100,000, 125,000, 135,000, 150,000, 165,000, 175,000, 180,000, 195,000, 200,000 or more and all ranges and subranges there between, for example a tensile strength range of 6,500 to 200,000 psi.

Examples of tensile strength modifiers or modifying agents include, but are not limited to, fats (e.g., hydrogenated or non-hydrogenated vegetable oils, animal fats), waxes (e.g., microcrystalline wax, bees wax), plasticizers/emulsifiers (e.g., mineral oil, fatty acids, mono- and diglycerides, triacetin, glycerin, acetylated monoglycerides, glycerol rosin monostearate esters), low and high molecular weight polymers (e.g., polypropylene glycol, polyethylene glycol, polyisobutylene, polyethylene, polyvinylacetate) and the like, and combinations thereof. Plasticizers may also be referred to as softeners.

Thus, by employing tensile strength modifiers, the overall tensile strength of the delivery system can be adjusted or altered in such a way that a preselected tensile strength is obtained for the corresponding desired release rate of the active component from an edible composition based on a comparison with a standard.

The tensile strength of the delivery system may be selected from relatively high tensile strengths when a relatively slow rate of release is desired and relatively lower tensile strengths when a faster rate of release is desired. Thus, when employing a tensile strength of 50,000 for a delivery system, the release rate of the active component, will generally be lower than the release rate of the active component in a delivery system having a tensile strength of 10,000 psi regardless of the type of encapsulating material (e.g. polyvinyl acetate) chosen as long as the hydrophobicity of the encapsulations is kept consistently similar or identical.

In a one embodiment, the encapsulating material is polyvinyl acetate. A representative example of a polyvinyl acetate product suitable for use as an encapsulating material in the present invention is Vinnapas® B100 material sold by Wacker Polymer Systems of Adrian, Michigan. A delivery system utilizing polyvinyl acetate may be prepared by melting a sufficient amount of polyvinyl acetate at a temperature of about 65° to 120°C for a short period of time, e.g., 5 minutes before adding to the continuous mixer. Alternatively, the polyvinyl acetate can be added to the mixer in solid form and melted in the mixer by, e.g., conveying through conveying region 26 before the addition of the active component(s). The melt temperature will depend on the type and tensile strength of the polyvinyl acetate encapsulating material where higher tensile strength materials will generally melt at higher temperatures. Once the encapsulating material is melted, a suitable amount of the active component (e.g., high intensity sweetener such as aspartame) is added to the mixer and blended into the molten mass thoroughly for an additional short period of mixing. The resulting mixture is a semi-solid mass, which is can be cooled (e.g., at 0°C) after exiting the mixer to obtain a solid, and then ground to a U.S. Standard sieve size of from about 30 to 200 (600 to 75 microns). The tensile strength of the resulting delivery system can readily be tested according to ASTM-D638 after molding the encapsulations in required size and shape.

The selection of a suitable encapsulating material will also depend in part on the type and amount of the active component and the presence of other additives or ingredients. Plasticizers or softeners as well as fats and oils, for example, act as "tensile strength modifying agents" and may be incorporated into the delivery system and particularly into the encapsulating material to modify the tensile strength of the resulting delivery system. The above mentioned additives may be added to the encapsulating material during the molten state. The amount of additives used in the delivery system of the present invention will of course vary according to the desired tensile strength can range up to 40% by weight based on the total weight of the delivery system.

In formulating the delivery system to have a predetermined tensile strength and a preselected hydrophobic encapsulating material, the active component can be entirely encapsulated within the encapsulating material or incompletely encapsulated within the encapsulating material provided the resulting tensile strength of the delivery system meets the criteria set forth hereinabove. The incomplete encapsulation can be accomplished by modifying and/or adjusting the manufacturing process to get partial coverage of the active component.

The addition of fats and oils to the mixer can have two effects on the delivery system. The first effect is observed at lower concentrations, i.e. up to 5% by weight, including up to 4.7, up to 4.5, up to 4.25, up to 4.0, up to 3.5, up to 3.0, up to 2.5, up to 2.25, up to 2.0, up to 1.75, up to 1.5, up to 1.0 and all values and ranges therebetween, wherein the fats and/or oils either maintain or increase the tensile strength of the delivery system. At higher concentrations (i.e., typically above 5% by weight), the fats and/or oils tend to reduce the tensile strength of the delivery system. Even with such unusual or non-linear effects on the tensile strength of the delivery system, a suitable delivery system with the desired release of the active component can be prepared based on sample delivery systems having known release rates for the active component. Thus, such fats and/or oils can be added to the mixer as noted in the description hereinabove.

In some instances, some of the active components encapsulated within the encapsulating material may be miscible with the encapsulating material. For example, polyvinylacetate is one type of encapsulating material that can be used in the present invention. Some components, such as flavor which are short or medium chain esters, may interact with the polyvinylacetate (PVA) and thereby reduce the effectiveness of the controlled and/or delayed release profile of the active component.

Therefore, in one embodiment, by itself or combined with the other embodiments described herein, the active component can be coated with a "coating material" that is not miscible or at least less miscible relative to its miscibility with the encapsulating material. The active component can be coated with the coating material prior to or concurrently with its encapsulation with the encapsulating material.

The coating material can reduce the miscibility of the active component with the encapsulating material at least 5%, preferably 25 %, more preferably at least 50%, including, 10, 15, 20, 30, 40, 60, 70, 75, 80, 85, 90, 95% or more relative to the miscibility of the active component which is not coated by the coating material.

In one embodiment, the material used to coat the active component is a water soluble and/or hydrophilic material. Non-limiting examples of suitable coating materials include, gum Arabic, cellulose, modified cellulose, gelatin, polyols (eg., sorbitol, maltitol), cyclodextrin, zein, polyvinylalcohol, polymethylmethacrylate, and polyurethane. Mixtures of various coating materials may also be used.

The coating thickness will vary depending on starting particle size and shape of the active material as well as the desired weight percent coating level. In accordance with the present invention, the coating thickness is preferably from about 1 to about 200 microns, including 10, 20, 30, 40, 50, 60, 70, 80, 90, 100, 110, 120, 130, 140, 150, 160, 170, 180 and 190 microns and all values and ranges there between, for example, the thickness of coating material can be from about 10 to about 50 microns and 20 to 54 % by weight.

In addition to providing a barrier stability that can reduce and/or eliminate the miscibility of the active component, the coating material used in the present invention may also have good film forming properties which facilitates the formation of a barrier between the active component and the encapsulating material. Film forming properties as used herein means that the coating materials, after dissolution in at least one solvent (such as, for example, water and/or organic solvents), leaves a film on the active component to which it is applied, for example, once the at least one solvent evaporates, absorbs and/or dissipates on the active component. Furthermore, when the coating material is used in the preparation of edible compositions, such as chewing gum, one of ordinary skill in the art recognizes that the coating material should be chosen based on its taste, shelf life, stickiness, resistance to microbial growth, and other common criteria for selecting ingredients for consumption.

The active component can be coated with the coating material by applying the coating material to the active component using a pan, spray, batch, and/or continuous processes typically used to coat materials. In one embodiment, the coating material is dissolved or dispersed in a solvent to facilitate coating on the active component. The coating material can be delivered using conventional methods of coating substrates. In a preferred method of coating, a fluidized bed technique is employed which is described, for example, in U.S. Patent no. 3,196,827, the relevant contents of which are incorporated herein by reference.

In a further embodiment, by coating the active component and encapsulating the active component according to the description provided herein, a longer shelf life of the edible compositions can be attained. As used herein, shelf life is an indicia of the stability of the components of the edible compositions containing the active component. Using flavorants and/or sweeteners for illustration, this increase in shelf life can be assessed by determining the perceived flavor and/or sweetness of the flavorant and/or sweetener contained in the composition. In some embodiments, when using a coating material to coat the active component a 5% increase in shelf life relative to a similar product in which the active component has not been coated with the barrier material can be achieved, including 10, 20, 30, 40, 50, 60, 70, 80, 90, 100% or more, as well as all values and ranges there between, increased shelf life. In another embodiment, the longer shelf life can be correlated to the time of storage after manufacture, for example at 10 weeks the shelf life the composition containing the coated active component will demonstrate a 50%, 75%, 80%, or 90% improvement relative to a similar composition but not containing an active component coated with a coating material according to the invention described herein. In a further example, at 24 weeks of storage, the coated active component will show an 80 to 90% improvement relative to a similar composition but not containing the active component coated with a coating material as according to the invention described herein.

In some embodiments, the delivery system may be in the form of a powder or granules. The particle size, generally, can vary and not have a significant effect on the function of the present invention. In one embodiment, the average particle size is desirably selected according to the desired rate of release and/or mouthfeel (i.e., grittiness) and the type of carrier incorporated in the edible composition. Thus, in certain embodiments of the present invention, the average particle size is from about 75 to about 600 microns, including 100, 110, 140, 170, 200, 230, 260, 290, 320, 350, 370 and all values and ranges there between. As the values are an average one will appreciate within a given sample of powder or granules, there may be particles with sizes greater and/or less than the numerical values provided. In one embodiment of the invention, where the delivery system is incorporated into a chewing gum the particle size can be less than 600 microns.

The at least one active component incorporated into the delivery system manufactured according to the processes described herein include, for example, a sweetener, such as a high-intensity sweetener, an acid, e.g., a food grade acid, a flavorant, a pharmaceutical, a therapeutic agent, a vitamin, a mineral, a breath freshener, a tooth whitener or cleaner, a cooling agent, a warming agent, a sensate, throat-soothing agents, spices, caffeine, drugs, etc. Combinations of these active components can be included in the same or different delivery systems. Such components may be used in amounts sufficient to achieve their intended effects.

A variety of well known cooling agents may be employed. For example, among the useful cooling agents are included menthol, xylitol, menthane, menthone, ketals, menthone ketals, menthone glycerol ketals, substituted p-menthanes, acyclic carboxamides, substituted cyclohexanamides, substituted cyclohaxane carboxamides, substituted ureas and sulfonamides, substituted menthanols, hydroxymethyl and hydroxymethyl derivatives of p-menthane, 2-mercapto-cyclo-decanone, 2-isoprpanyl-5-methylcyclohexanol, hydroxycarboxylic acids with 2-6 carbon atoms, cyclohexanamides, menthyl acetate, menthyl lactate, menthyl salicylate, N,2,3-trimethyl-2-isopropyl butanamide (WS-23), N-ethyl-p-menthane-3-carboxamide (WS-3), menthyl succinate, 3,1-menthoxypropane 1,2-diol, among others. These and other suitable cooling agents are further described in the following U.S. patents, all of which are incorporated in their entirety by reference hereto: U.S. 4,230,688; 4,032,661; 4,459,425; 4,136,163; 5,266,592; 6,627,233.

Examples of food grade acids which can be used include acetic acid, adipic acid, ascorbic acid, butyric acid, citric acid, formic acid, fumaric acid, glyconic acid, lactic acid, phosphoric acid, malic acid, oxalic acid, succinic acid, tartaric acid and others.

Warming components may be selected from a wide variety of compounds known to provide the sensory signal of warming to the user. These compounds offer the perceived sensation of warmth, particularly in the oral cavity, and often enhance the perception of flavors, sweeteners and other organoleptic components. Among the useful warming compounds included are vanillyl alcohol n-butylether (TK-1000) supplied by Takasago Perfumary Company Limited, Tokyo, Japan, vanillyl alcohol n-propylether, vanillyl alcohol isopropylether, vanillyl alcohol isobutylether, vanillyl alcohol n-aminoether, vanillyl alcohol isoamyleather, vanillyl alcohol n-hexyleather, vanillyl alcohol methylether, vanillyl alcohol ethyleather, gingerol, shogaol, paradol, zingerone, capsaicin, dihydrocapsaicin, nordihydrocapsaicin, homocapsaicin, homodihydrocapsaicin, ethanol, isopropyl alcohol, iso-amylalcohol, benzyl alcohol, glycerine, and combinations thereof.

The sensation of warming or cooling effects may be prolonged with the use of a hydrophobic sweetener as described in U.S. Patent Application Publication 2003/0072842 A1 which is incorporated in its entirety herein by reference. For example, such hydrophobic sweeteners include those of the formulae I-XI referenced therein. Perillartine may also be added as described in U.S. Patent No. 6,159,509 also incorporated in its entirety herein by reference.

The breath freshening agents may include in addition to the flavors and cooling agents described hereinabove, a variety of compositions with odor controlling properties. These may include, without limitation, cyclodextrin and magnolia bark extract. The breath freshening agents may further be encapsulated to provide a prolonged breath freshening effect. Examples of malodor-controlling compositions are included in U.S. Patent No. 5,300,305 to Stapler et al. and in U.S. Patent Application Publication Nos. 2003/0215417 and 2004/0081713 which are incorporated in their entirety herein by reference.

As described above, a variety of oral care products may also be included in some embodiments of chewing gums. These may include tooth whiteners, stain removers and anticalculus agents. Examples of these include, but are not limited to hydrolytic agents including proteolytic enzymes, abrasives such as hydrated silica, calcium carbonate, sodium bicarbonate and alumina, other active stain-removing components such as surface-active agents, such as anionic surfactants such as sodium stearate, sodium palminate, sulfated butyl oleate, sodium oleate, salta of fumaric acid, glycerol, hydroxylated lecithin, sodium lauryl sulfate and chelators such as polyphosphates, which are typically employed in dentifrice compositions as tartar control ingredients. Also included are tetrasodium pyrophosphate and sodium tri-polyphosphate, sodium tripolyphosphate, xylitol, hexametaphosphate, and an abrasive silica. Further examples are included in the following U.S. Patents which are incorporated in their entirety herein by reference: U.S. Patent Nos. 5,227,154, 5,378,131 and 6,685,916.

A variety of drugs, including medications, herbs, and nutritional supplements may also be included in the gum formulations. Examples of useful drugs include ace-inhibitors, antianginal drugs, anti-arrhythmias, anti-asthmatics, anti-cholesterolemics, analgesics, anesthetics, anti-convulsants, anti-depressants, anti-diabetic agents, anti-diarrhea preparations, antidotes, anti-histamines, anti-hypertensive drugs, anti-inflammatory agents, anti-lipid agents, anti-manics, anti-nauseants, anti-stroke agents, anti-thyroid preparations, anti-tumor drugs, anti-viral agents, acne drugs, alkaloids, amino acid preparations, anti-tussives, anti-uricemic drugs, anti-viral drugs, anabolic preparations, systemic and non-systemic anti-infective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil

citrate, which is currently marketed as Viagra^{®}, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocryptine or nicotine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of other active ingredients include antacids, H2-antagonists, and analgesics. For example, antacid dosages can be prepared using the ingredients calcium carbonate alone or in combination with magnesium hydroxide, and/or aluminum hydroxide. Moreover, antacids can be used in combination with H2-antagonists.

Analgesics include opiates and opiate derivatives, such as Oxycontin, ibuprofen, aspirin, acetaminophen, and combinations thereof that may optionally include caffeine.

Other drug ingredients for use in embodiments include anti-diarrheals such as immodium AD, anti-histamines, anti-tussives, decongestants, vitamins, and breath fresheners. Also contemplated for use herein are anxiolytics such as Xanax; anti-psychotics such as clozaril and Haldol; non-steroidal anti-inflammatories (NSAID's) such as ibuprofen, naproxen sodium, Voltaren and Lodine, anti-histamines such as Claritin, Hismanal, Relafen, and Tavist; anti-emetics such as Kytril and Cesamet; bronchodilators such as Bentolin, Proventil; anti-depressants such as Prozac, Zoloft, and Paxil; anti-migraines such as Imigra, ACE-inhibitors such as Vasotec, Capoten and Zestril; anti-Alzheimer's agents, such as Nicergoline; and CaH-antagonists such as Procardia, Adalat, and Calan.

H2-antagonists which can be used include cimetidine, ranitidine hydrochloride, famotidine, nizatidien, ebrotidine, mifentidine, roxatidine, pisatidine and aceroxatidine

Active antacid ingredients include, but are not limited to, aluminum hydroxide, dihydroxyaluminun aminoacetate, aminoacetic acid, aluminum phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminum mono-ordibasic calcium phosphate, tricalcium phosphate, potassium bicarbonate, sodium tartrate, sodium bicarbonate, magnesium aluminosilicates, tartaric acids and salts.

A variety of other nutritional supplements may also be included, such as vitamin or mineral as mentioned above. For example, vitamin A, vitamin C, vitamin D, vitamin E, vitamin K, vitamin B6, vitamin B12, thiamine, riboflavin, biotin, folic acid, niacin, pantothenic acid, sodium, potassium, calcium, magnesium, phosphorus, sulfur, chlorine, iron, copper, iodine, zinc, selenium, manganese, choline, chromium, molybdenum, fluorine, cobalt and combinations thereof, may be used.

Examples of nutritional supplements are set forth in U.S. Patent Application Publication Nos. 2003/0157213 A1, 2003/0206993 and 2003/0099741 A1 which are incorporated in their entirety herein by reference.

Various herbs may also he included such as those with various medicinal or dietary supplement properties. Herbs are generally aromatic plants or plant parts that can be used medicinally or for flavoring. Suitable herbs can be used singly or in various mixtures. Examples include Echinacea, Goldenseal, Calendula, Aloe, Blood Root, Grapefruit Seed Extract, Black Cohosh, Cranberry, Ginko Biloba, St. John's Wort, Evening Primrose Oil, Yohimbe Bark, Green Tea, Maca, Bilberry, Lutein, and combinations thereof.

Flavorants which may be used include those flavors known to the skilled artisan, such as natural and artificial flavors. These flavorings may be chosen from synthetic flavor oils and flavoring aromatics and/or oils, oleoresins and extracts derived from plants, leaves, flowers, fruits, and so forth, and combinations thereof. Nonlimiting representative flavor oils include spearmint oil, cinnamon oil, oil of wintergreen (methyl salicylate), peppermint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, and fruit essences including apple, pear, peach, grape, blueberry, strawberry, raspberry, cherry, plum, pineapple, apricot and so forth. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture. Flavors may also provide breath freshening properties, particularly the mint flavors when used in combination with the cooling agents, described herein below.

Other useful flavorings include aldehydes and esters such as cinnamyl acetate, cinnamaldehyde, citral diethylacetal, dihydrocarvyl acetate, eugenyl formate, p-methylamisol, and so forth may be used. Generally any flavoring or food additive such as those described in Chemicals Used in Food Processing, publication 1274, pages 63-258, by the National Academy of Sciences, may be used. This publication is incorporated herein by reference. This may include natural as well as synthetic flavors.

Further examples of aldehyde flavorings include but are not limited to acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6-dimethyl-5-heptenal, .e., melonal (melon), 2,6-dimethyloctanal (green fruit), and 2-dodecenal (citrus, mandarin), cherry, grape, blueberry, blackberry, strawberry shortcake, and mixtures thereof.

The sweeteners used may be selected from a wide range of materials including water-soluble sweeteners, water-soluble artificial sweeteners, water-soluble sweeteners derived from naturally occurring water-soluble sweeteners, dipeptide based sweeteners, and protein based sweeteners, including mixtures thereof. Without being limited to particular sweeteners, representative categories and examples include:

(a) water-soluble sweetening agents such as dihydrochalcones, monellin, steviosides, glycyrrhizin, dihydroflavenol, sugar alcohols such as sorbitol, mannitol, maltitol, xylitol, erithrytol, isomalt, and L-aminodicarboxylic acid aminoalkenoic acid ester amides, such as those disclosed in U.S. Pat. No. 4,619,834, which disclosure is incorporated herein by reference, and mixtures thereof;

(b) water-soluble artificial sweeteners such as soluble saccharin

salts, i.e., sodium or calcium saccharin salts, cyclamate salts, acesulfame salts, such as the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and mixtures thereof;

(c) dipeptide based sweeteners, such as L-aspartic acid derived sweeteners, such as L-aspartyl-L-phenylalanine methyl ester (Aspartame) and materials described in U.S. Pat. No. 3,492,131, L-alphaaspartyl-N-(2,2,4,4-tetramethyl-3-thietanyl)-D-alaninamide hydrate (Alitame), methyl esters of L-aspartyl-L-phenylglycerine and L-aspartyl-L-2,5-dihydrophenyl-glycine, L-aspartyl-2,5-dihydro-L-phenylalanine; L-aspartyl-L-(1-cyclohexen)-alanine, neotame, and mixtures thereof;

(d) water-soluble sweeteners derived from naturally occurring water-soluble sweeteners, such as stevosides, chlorinated derivatives of ordinary sugar (sucrose), e.g., chlorodeoxysugar derivatives such as derivatives of chlorodeoxysucrose or chlorodeoxygalactosucrose, known, for example, under the product designation of Sucralose; examples of chlorodeoxysucrose and chlorodeoxygalactosucrose derivatives include but are not limited to: 1-chloro-1'-deoxysucrose; 4-chloro-4-deoxy-alpha-D-galactopyranosyl-alpha-D-fructofuranoside, or 4-chloro-4-deoxygalactosucrose; 4-chloro-4-deoxy-alpha-D-galac-topyranosyl-1-chloro-1-deoxy-beta-D-fructo-furanoside, or 4, 1'-dichloro-4, 1'-dideoxygalactosucrose; 1',6'-dichlorol',6'-dideoxysucrose; 4-chloro-4-deoxy-alpha-D-galactopyranosyl-1,6-dichloro-1,6-dideoxy-beta-D- fructofuranoside, or 4,1',6'-trichloro-4,1',6'-trideoxygalactosucrose; 4,6-dichloro-4,6-dideoxy-alpha-D-galactopyranosyl-6-chloro-6-deoxy-beta-D- fructofuranoside, or 4,6,6'-trichloro-4,6,6'-trideoxygalactosucrose; 6,1',6'-trichloro-6,1',6'-trideoxysucrose; 4,6-dichloro-4,6-dideoxy-alpha-D-galacto-pyranosyl-1,6-dichloro-1,6-dideoxy-beta-D-fructoranoside, or

4,6,1',6'-tetrachloro4,6,1',6'-tetradeoxygalacto-sucrose; and 4,6,1',6'-tetradeoxy-sucrose, and mixtures thereof;

(e) protein based sweeteners such as thaumaoccous danielli (Thaumatin I and II), talin; and

(f) amino acid based sweeteners.

The intense sweetening agents may be used in many distinct physical forms well-known in the art to provide an initial burst of sweetness and/or a prolonged sensation of sweetness. Without being limited thereto, such physical forms include free forms, such as spray dried, powdered, beaded forms, encapsulated forms, and mixtures thereof In one embodiment, the sweetener is a high intensity sweetener such as aspartame, sucralose, neotame, and acesulfame potassium (Ace-K).

The active component (e.g., sweetener), which is part of the delivery system, may be used in amounts necessary to impart the desired effect associated with use of the active component (e.g., sweetness). With respect to their presence in the delivery system, the active components may be present in amounts of from about 1% to 70% by weight based on the total weight of the delivery system, including 5, 10, 15, 20, 25, 30, 35, 40, 45, 50, 55, 60, 65% by weight, and all values and ranges there between, for example, from about 10% to 40% by weight based on the total weight of the delivery system. For typical edible compositions including chewing gum compositions, confectionery compositions and beverage compositions, the sweeteners may be present in amounts of from about 0.1% to 6% by weight based on the total weight of the edible composition, including 0.5, 1, 2, 3, 4, 5 % by weight and all values and subranges there between, for example, 0.5% to 3% by weight. The active component especially when the active component is a sweetener may also be present in the edible composition in free form depending on the release profile desired.

In another aspect, there is provided a method of manufacturing edible compositions which comprise the present delivery system and a carrier in an amount appropriate to accommodate the delivery system. The term "carrier" as used herein refers to an orally acceptable vehicle such as the soluble and insoluble components of a chewing gum composition capable of being mixed with the delivery system, and which will not cause harm to warm-blooded animals including humans. The carriers further include those components of the composition that are capable of being commingled without significant interaction with the delivery system.

In a one embodiment, the edible composition is a chewing gum composition having prolonged release (e.g., typically at least 15 minutes) of the active component. The chewing gum composition comprises a chewing gum base and the delivery system of the present invention that comprises an encapsulating material and at least one encapsulated active component such as, for example, a sweetener or a flavorant. The delivery system is present in amounts from about 0.2% to 10% by weight based on the total weight of the chewing gum composition, including 0.5, 1.0, 2.0, 3.0, 4.0, 5.0, 6.0, 7.0, 8.0, 9.0 % by weight including all values and subranges there between, for example, from about 1% to 5% by weight.

The present invention may be incorporated with a variety of processes for preparing chewing gum compositions as known in the art. Such chewing gum compositions may be and include a variety of different formulations that are typically used to make chewing gum products. Typically, a chewing gum composition contains a chewable gum base portion, which is essentially free of water and is water insoluble and a water soluble bulk portion.

The water soluble portion is generally released from the gum base portion over a period of time during chewing. The gum base portion is retained in the mouth throughout the chewing. The water insoluble gum base generally comprises elastomers, elastomer solvents, plasticizers, waxes, emulsifiers, and inorganic fillers. Plastic polymers such as polyvinyl acetate, which behave somewhat as plasticizers, are also included. Other plastic polymers that may be used include polyvinyl laurate, crosslinked polyvinyl pyrrolidone and polyhydroxy alkanoates.

The elastomers may constitute from about 5% to 95% by weight of the gum base. In another embodiment, the elastomers may constitute from about 10% to 70% by weight of the gum base and in another embodiment, 15% to 45% by weight of the gum base. Examples of elastomers include synthetic elastomers such as polyisobutylene, polybutylene, isobutylene-isoprene co-polymers, styrene-butadiene co-polymers, polyvinyl acetate and the like. Elastomers may also include natural elastomers such as natural rubber as well as natural gums such as jelutong, lechi caspi, perillo, massaranduba balata, chicle, gutta hang kang or combinations thereof. Other elastomers are known to those of ordinary skill in the art.

Elastomer plasticizers modify the finished gum firmness when used in the gum base. Elastomer plasticizers are typically present in an amount up to 75% by weight of the gum base. In another embodiment, the elastomer plasticizers are present in an amount of from about 5% to 45% by weight of the gum base and in another embodiment from about 10% to 30% by weight of gum base. Examples of elastomer plasticizers include natural rosin esters such as glycerol ester of partially hydrogenated rosin, glycerol ester of tall oil rosin, pentaerythritol esters of partially hydrogenated rosin, methyl and partially hydrogenated methyl esters of rosin, and the like. Synthetic elastomer plasticizers such as terpene resins may also be employed in gum base composition.

Waxes include synthetic and naturally occurring waxes such as polyethylene, bees wax, carnauba and the like. Petroleum waxes such a paraffin may also be used. The waxes may be present in the amount up to 30% by weight of the gum base. Waxes aid in the curing of the finished gum and help improve the release of flavor and may further extend the shelf life of the product.

Elastomer solvents are often resins such as terpene resins. Plasticizers, sometimes referred to as softeners, are typically fats and oils, including tallow, hydrogenated vegetable oils, and cocoa butter.

Gum base typically also includes a filler component. The filler component modifies the texture of the gum base and aid processing. Examples of such fillers include magnesium and aluminum silicates, clay, alumina, talc, titanium oxide, cellulose polymers, and the like. Fillers are typically present in the amount of from 1% to 60% by weight.

Emulsifiers, which sometimes also have plasticizing properties, include glycerol monostearate, lecithin, and glycerol triacetate. Further, gum bases may also contain optional ingredients such as antioxidants, colors, and flavors.

The insoluble gum base may be present in the amount of from about 5% to 95% by weight of the chewing gum. In one embodiment, the insoluble gum base may present in the amount of from about 10% to 50% by weight of the gum base, and in another embodiment from about 20% to 40% by weight of the gum base.

Softeners are added to the chewing gum in order to optimize the chewability and mouth feel of the gum. Softeners, also known in the art as plasticizers or plasticizing agents, is generally present in amounts from about 0.5% to 15% by weight based on the total weight of the chewing gum composition. Softeners contemplated by the present invention include, for example, lecithin. Further, aqueous sweetener solutions such as those containing sorbitol, hydrogenated starch hydrolysate, corn syrup, and combinations thereof may be used as softeners and binding agents in the gum.

The chewing gum compositions may be coated or uncoated and be in the form or slabs, sticks, pellets, balls and the like. The composition of the different forms of the chewing gum compositions will be similar but may vary with regard to the ratio of the ingredients. For example, coated gum compositions may contain a lower percentage of softeners. Pellets and balls have a small chewing gum core, which is then coated with either a sugar solution or a sugarless solution to create a hard shell. Slabs and sticks are usually formulated to be softer in texture than the chewing gum core.

In accordance with one aspect of the chewing gum composition, the delivery system is added during the manufacture of the chewing gum composition. In another aspect of the present invention, the delivery system is added as one of the last steps, for example, the last step in the formation of the chewing gum composition.

Applicants have determined that this process modification incorporates the delivery system into the gum composition without materially binding the delivery system therein such as may occur if the delivery system is mixed directly with the gum base. Thus, the delivery system, while only loosely contained within the gum composition can more effectively release the active component therefrom during a typical chewing operation. Thus, a material portion of the delivery system is free of the gum base and the corresponding ingredients of the chewing gum.

Incorporation of delivery systems into different stages of mixing the ingredients for the gum may be used to provide different delivery rates, e.g., early and late, and as such the manufacturing process can be adjusted accordingly.

Coating techniques for applying a coating for a chewing gum composition such as pan and spray coating are well known. In one embodiment, coating with solutions adapted to build a hard candy layer can be employed. Both sugar and sugar alcohols may be used for this purpose together with high intensity sweeteners, colorants, flavorants and binders.

Other components may be added in minor amounts to the coating syrup and include moisture absorbing compounds, anti-adherent compounds, dispersing agents and film forming agents. The moisture absorbing compounds suitable for use in the coating syrups include mannitol or dicalcium phosphate. Examples of useful anti-adherent compounds, which may also function as a filler, include talc, magnesium trisilicate and calcium carbonate. These ingredients may be employed in amounts of from about 0.5% to 5% by weight of the syrup. Examples of dispersing agents, which may be employed in the coating syrup, include titanium dioxide, talc or other anti-adherent compounds as set forth above.

The coating syrup is usually heated and a portion thereof deposited on the cores. Usually a single deposition of the coating syrup is not sufficient to provide the desired amount or thickness of coating and second, third or more coats of the coating syrup may be applied to build up the weight and thickness of the coating to desired levels with layers allowed to dry in-between coats.

A method of preparing a chewing gum composition is provided by sequentially adding the various chewing gum ingredients including the delivery system of the present invention to any commercially available mixer known in the art. After the ingredients have been thoroughly mixed, the gum base is discharged from the mixer and shaped into the desired form such as by rolling into sheets and cutting into sticks, extruding into chunks, or casing into pellets.

Generally, the ingredients are mixed by first melting the gum base and adding it to the running mixer. The base may also be melted into the mixer itself. Colors or emulsifiers may also be added at this time. A softener may be added to the mixer at this time, along with syrup and a portion of the bulking agent. Further parts of the bulking agent are then added to the mixer. Flavorants are typically added with the final portion of the bulking agent. Finally, the delivery system exhibiting a predetermined tensile strength is added to the resulting mixture. Other optional ingredients are added in the batch in a typical fashion, well known to those of ordinary skill in the art.

The entire mixing procedure typically takes from five to fifteen minutes, but longer mixing times may be required. Those skilled in the art will recognize that many variations of the above-described procedure may be follows.

After the ingredients are mixed, the gum mass may be formed into a variety of shapes and products. For example, the ingredients may be formed into pellets or balls and used as cores to make a coated chewing gum product. However, any type of chewing gum product can be utilized with the present invention.

If a coated product is desired, the coating may contain ingredients such as flavorants, artificial sweeteners, dispersing agents, coloring agents, film formers and binding agents. Flavorants contemplated by the present invention, include those commonly known in the art such as essential oils, synthetic flavors, or mixtures thereof, including but are not limited to, oils derived from plants and fruits such as citrus oils, fruit essences, peppermint oil, spearmint oil, other mint oils, clove oil, oil of wintergreen, anise and the like. The flavorants may also be added to the coating syrup in an amount such that the coating may be present in amounts of from about 0.2% to 1.2% by weight flavoring agent. In another embodiment, the coating may be present in amounts, and more preferably from about 0.7% to 1.0% by weight flavoring agent.

Dispersing agents are often added to syrup coatings for the purpose of whitening and tack reduction. Dispersing agents contemplated by the present invention to be employed in the coating syrup include titanium dioxide, talc, or any other anti-stick compound. The dispersing agent may be added to the coating syrup in an amount such that the coating contains from about 0.1 % to 1.0%, including 0.2, 0.3, 0.4, 0.5, 0.6, 0.7, 0.8, 0.9 and all values and ranges there between, for example, from about 0.3% to 0.6% by weight of the agent.

Coloring agents may be added directly to the coating syrup in dye or lake form. Coloring agents contemplated by the present invention include food quality dyes. Film formers may be added to the coating syrup include methylcellulose, carboxymethyl cellulose, ethyl cellulose, hydroxyethyl cellulose, and the like or combinations thereof. Binding agents may be added either as an initial coating on the chewing gum center or may be added directly to the coating syrup. Binding agents contemplated by the present invention include gum arabic, gum talha, gelatin, vegetable gums, and the like. The binding agents, when added to the coating syrup, are typically added in amounts from about 0.5% to 10% by weight.

The present invention further encompasses confectionery compositions containing the delivery system of the present invention. Confectionery compositions include, for example, compressed tablets such as mints, hard boiled candies, chocolates, chocolate containing products, nutrient bars, nougats, gels, centerfill confections, fondants, panning goods, consumable thin films and other compositions falling within the generally accepted definition of confectionery compositions.

Confectionery compositions in the form of pressed tablets such as mints may generally be made by combining finely sifted sugar or sugar substitute, flavoring agent (e.g. peppermint flavor) bulking agent such as gum arabic, and an optional coloring agent. The flavoring agent, bulking agent are combined and then gradually the sugar or sugar substitute are added along with a coloring agent if needed.

The product is then granulated by passing through a seize of desired mesh size (e.g., 12 mesh) and then dried typically at temperatures of from about 55°C to 60°C. The resulting powder is fed into a tableting machine fitted with a large size punch and the resulting pellets are broken into granules and then pressed.

High boiled candies typically contain sugar or sugar substitute, glucose, water, flavoring agent and optional coloring agent. The sugar is dissolved in the water and glucose is then added. The mixture is brought to a boil. The resulting liquid to which may previously have been added a coloring agent is poured onto an oiled slab and cooled. The flavoring agent are then added and kneaded into the cooled mass. The resulting mixture is then fed to a drop roller assembly known in the art to form the final hard candy shape.

A nougat composition typically includes two principal components, a high boiled candy and a frappe. By way of example, egg albumen or substitute thereof is combined with water and whisked to form a light foam. Sugar and glucose are added to water and boiled typically at temperatures of from about 130°C to 140°C and the resulting boiled product is poured into a mixing machine and beat until creamy.

The beaten albumen and flavoring agent are combined with the creamy product and the combination is thereafter thoroughly mixed.

Further details regarding the preparation of confectionery compositions can be found in Skuse's Complete Confectioner (13th Edition) (1957) including pp. 41-71, 133-144, and 255-262; and Sugar Confectionery Manufacture (2nd Edition) (1995), E.B. Jackson, Editor, pp. 129-168, 169-188, 189-216, 218-234, and 236-258 each of which is incorporated herein by reference.

Except as otherwise noted, the amount of the ingredients incorporated into the compositions according to the present invention is designated as % by weight based on the total weight of the composition.

EXAMPLES

The following are examples of systems which can be processed according the method described herein. In each example, the temperature range of operation, the type of extruder and the speed at which the extruder is operated are described.

Example 1: Encapsulation of Sucralose using medium molecular weight PVA (MW ≈ 35,000 - 45,000)

PVA is added to a twin screw extruder operating at a screw speed of from about 100 to about 400 RPM in mixing zone 1 as shown in Fig. 1. The extruder is operated in a temperature range of 60-110 °C. The temperature of the processing is preferably kept as low as possible to avoid significant degradation in the sucralose being encapsulated. Fat is added to the extruder in mixing zone 4 and sucralose is added to mixing zone 6 as shown in Fig. 1. The ingredients are added at a flow rate ranging from 10 to 1500 lbs per hour.

| | |
|---|---|
| **Ingredient** | **Percent** |
| | |
| Polyvinyl Acetate | 77-97% |
| Fat | 0.5-13% |
| Sucralose | 1.0-45% |
| **Total** | **100.00%** |

In further examples of such formulations, the polyvinyl acetate can be added, for example, in amounts from 80-90% and 87-95%; the fat added, for example, in amounts of 1-10 and 3-5%; and the sucralose added, for example, in amounts of from 10-30 and 15-25%.

Example 2: Encapsulation of Aspartame using high molecular weight PVA (MW ≈80,000 -100,000)

PVA is added to a twin screw extruder operating at a screw speed of from about 100 to about 400 RPM in mixing zone 1 as shown in Fig. 1. The extruder is operated in a temperature range of 60-140 °C. Fat and glycerol monostearte are added to the extruder in mixing zone 4 and aspartame is added to mixing zone 6 as shown in Fig. 1. The ingredients are added at a flow rate ranging from 10 to 1500 lbs per hour.

| **Ingredient** | **Percent** |
|---|---|
| | |
| Polyvinyl Acetate | 55-75% |
| Fat | 0.5-6% |
| Glycerol Monostearte | 0.5-6% |
| Aspartame | 1-45% |
| **Total** | **100.00%** |

In further examples of such formulations, the polyvinyl acetate can be added, for example, in amounts from 60-70 and 65-75%; the fat added, for example, in amounts of 1-5 and 3.5-4%; the glycerol monostearate added, for example, in amounts of from 1-3 and 1.25-2 % and the aspartame added, for example, in amounts of from 10-34 and 15-25%.

Example 3: Encapsulation of Acesulfame K using high molecular weight PVA (M W ≈80,000 -100,000)

PVA is added to a twin screw extruder operating at a screw speed of from about 100 to about 400 RPM in mixing zone 1 as shown in Fig. 1. The extruder is operated in a temperature range of 60-140 °C. Fat and glycerol monostearte are added to the extruder in mixing zone 4 and acesulfame K is added to mixing zone 6 as shown in Fig. 1. The ingredients are added at a flow rate ranging from 10 to 1500 lbs per hour.

| | |
|---|---|
| **Ingredient** | **Percent** |
| | |
| Polyvinyl Acetate | 55-75% |
| Fat | 0.5-6% |
| Glycerol Monostearte | 0.5-6% |
| Acesulfame K | 1-45% |
| **Total** | **100.00%** |

In further examples of such formulations, the polyvinyl acetate can be added, for example, in amounts from 60-75 and 65-70%; the fat added, for example, in amounts of 1-4 and 2-3.75%; the glycerol monostearate added, for example, in amounts of from 1-4 and 2-3 % and the acesulfame K added, for example, in amounts of from 10-30 and 15-25%.

Example 4: Encapsulation of Neotame using low molecular weight PVA (MW ≈ 10,000 - 15,000)

PVA is added to a twin screw extruder operating at a screw speed of from about 100 to about 400 RPM in mixing zone 1 as shown in Fig. 1. The extruder is operated in a temperature range of 60-140 °C. Fat and glycerol monostearte are added to the extruder in mixing zone 4 and neotame is added to mixing zone 6 as shown in Fig. 1. The ingredients are added at a flow rate ranging from 10 to 1500 lbs per hour.

| | |
|---|---|
| **Ingredient** | **Percent** |
| | |
| Polyvinyl Acetate | 80-95% |
| Fat | 0.5-6% |
| Glycerol Monostearte | 0.5-6% |
| Neotame | 1.0-45% |
| **Total** | **100.00%** |

In further examples of such formulations, the polyvinyl acetate can be added, for example, in amounts from 85-94 and 87-90%; the fat added, for example, in amounts of 1-4 and 2-3%; the glycerol monostearate added, for example, in amounts of from 1-5 and 2-3.5% and the neotame added, for example, in amounts of from 10-30 and 15-25%.

Example 5: Encapsulation of Aspartame using Poly(methyl methacrylate)

Poly(methyl methacrylate) is added to a twin screw extruder operating at a screw speed of from about 100 to about 400 RPM in mixing zone 1 as shown in Fig. 1. The extruder is operated in a temperature range of 60-140 °C. Fat and glycerol monostearte are added to the extrude in mixing zone 4 and aspartame is added to mixing zone 6 as shown in Fig. 1. The ingredients are added at a flow rate ranging from 10 to 1500 lbs per hour.

| | |
|---|---|
| **Ingredient** | **Percent** |
| | |
| Poly(methyl methacrylate) | 55-75% |
| Fat | 0.5-6% |
| Glycerol Monostearte | 0.5-6% |
| Aspartame | 1-45% |
| **Total** | **100.00%** |

In further examples of such formulations, the Poly(methyl methacrylate) can be added, for example, in amounts from 57-70 and 60-65%; the fat added, for example, in amounts of 1-5 and 2-3.7%; the glycerol monostearate added, for example, in amounts of from 1.25-5 and 2-4% and the aspartame added, for example, in amounts of from 10-30 and 15-25%.

Example 6: Encapsulation of Aspartame using Poly(Ethyl methacrylate)

Poly(Ethyl methacrylate) is added to a twin screw extruder operating at a screw speed of from about 100 to about 400 RPM in mixing zone 1 as shown in Fig. 1. The extruder is operated in a temperature range of 60-140 °C. Fat and glycerol monostearte are added to the extruder in mixing zone 4 and aspartame is added to mixing zone 6 as shown in Fig. 1. The ingredients are added at a flow rate ranging from 10 to 1500 lbs per hour.

| | |
|---|---|
| **Ingredient** | **Percent** |
| | |
| Poly(ethyl methacrylate) | 55-75% |
| Fat | 0.5-6% |
| Glycerol Monostearte | 0.5-6% |
| Aspartame | 1-45% |
| **Total** | **100.00%** |

In further examples of such formulations, the Poly(ethyl methacrylate) can be added, for example, in amounts from 57-70 and 60-65%; the fat added, for example, in amounts of 1-5 and 2-3.7%; the glycerol monostearate added, for example, in amounts of from 1.25-5 and 2-4% and the aspartame added, for example, in amounts of from 10-30 and 15-25%.

Examples 7: Encapsulation of Aspartame using Polyethylene

Polyethylene is added to a twin screw extruder operating at a screw speed of from about 85 to about 400 RPM in mixing zone 1 as shown in Fig. 1. The extruder is operated in a temperature range of 60-140 °C. Fat and glycerol monostearte are added to the extruder in mixing zone 4 and aspartame is added to mixing zone 6 as shown in Fig. 1. The ingredients are added at a flow rate ranging from 10 to 1500 lbs per hour.

| | |
|---|---|
| **Ingredient** | **Percent** |
| | |
| Polyethylene | 55-75% |
| Fat | 0.5-6% |
| Glycerol Monostearte | 0.5-6% |
| Aspartame | 1-45% |
| **Total** | **100.00%** |

In further examples of such formulations, the polyethylene can be added, for example, in amounts from 57-67 and 60-65%; the fat added, for example, in amounts of 1-5 and 2-3.7%; the glycerol monostearate added, for example, in amounts of from 1-5 and 2-4% and the aspartame added, for example, in amounts of from 10-30 and 15-25%.

Example 8: Encapsulation of Aspartame using Poly Co(Ethylene Vinyl Acetate) (7.5 % VA)

Poly Co(Ethylene Vinyl Acetate) (7.5 % VA) is added to a twin screw extruder operating at a screw speed of from about 85 to about 400 RPM in mixing zone 1 as shown in Fig. 1. The extruder is operated in a temperature range of 60-140 °C. Fat and glycerol monostearte are added to the extruder in mixing zone 4 and aspartame is added to mixing zone 6 as shown in Fig. 1. The ingredients are added at a flow rate ranging from 10 to 1500 lbs per hour.

| | |
|---|---|
| **Ingredient** | **Percent** |
| | |
| Poly Co (Ethylene Vinyl Acetate) (7.5 % VA) | 55-75% |
| Fat | 0.5-6% |
| Glycerol Monostearte | 0.5-6% |
| Aspartame | 1-45% |
| **Total** | **100.00%** |

In further examples of such formulations, the Poly Co (Ethylene Vinyl Acetate) (7.5 % VA) can be added, for example, in amounts from 57-67 and 60-65%; the fat added, for example, in amounts of 1-3.75 and 2-3%; the glycerol monostearate added, for example, in amounts of from 1.25-4 and 2-3% and the aspartame added, for example, in amounts of from 10-30 and 15-25%.

Example 9: Encapsulation of Aspartame using Poly Co(Vinylacetate Vinylpyrrolidone)

Poly Co(Vinylacetate Vinylpyrrolidone) is added to a twin screw extruder operating at a screw speed of from about 85 to about 400 RPM in mixing zone 1 as shown in Fig. 1. The extruder is operated in a temperature range of 60-140 °C. Fat and glycerol monostearte arc added to the extruder in mixing zone 4 and aspartame is added to mixing zone 6 as shown in Fig. 1. The ingredients are added at a flow rate ranging from 10 to 1500 lbs per hour.

| | |
|---|---|
| **Ingredient** | **Percent** |
| | |
| Poly Co(Vinylacetate Vinylpyrrolidone) | 65-85% |
| Fat | 0.5-6% |
| Glycerol Monostearte | 0.5-6% |
| Aspartame | 1-45% |
| **Total** | **100.00%** |

In further examples of such formulations, the Poly Co(Vinylacetate Vinylpyrrolidone) can be added, for example, in amounts from 67-80 and 70-75%; the fat added, for example, in amounts of 1-4 and 2-3%; the glycerol monostearate added, for example, in amounts of from 1.25-4.5 and 2-3.75% and the aspartame added, for example, in amounts of from 15-40 and 20-30%.

Obviously, numerous modifications and variations of the present invention are possible in light of the above teachings. It is therefore to be understood that within the scope of the appended claims, the invention may be practiced otherwise than as specifically described herein.

### Annex to Description

1. A method of manufacturing a delivery system comprising at least one active component encapsulated in an encapsulating material, the method comprising adding the encapsulating material and the at least one active component to a continuous mixer; mixing the encapsulating material with the at least one active component such that the at least one active component is at least partially encapsulated by the encapsulating material.
2. The method of item 1, wherein the at least one active component is completely encapsulated by the encapsulating material.
3. The method of item 1, wherein the temperature of the encapsulating material is maintained at a temperature to minimize degradation of the at least one active component.
4. The method of item 1, which further comprises feeding one or more ingredients selected from the group consisting of a fat, an emulsifier, a plasticizer, a softener, a low molecular weight polymer, a high molecular weight polymer, a wax, and a combination thereof into the first conveying region.
5. The method of item 1, further comprising adding at least a portion of the at least one active component to the continuous mixer with the encapsulating material.
6. The method of item 1, wherein the delivery system produced has a tensile strength of at least about 6,500 psi.
7. The method of item 1, wherein the delivery system produced has a tensile strength of at least about 10,000 psi.
8. The method of item 1, wherein the delivery system produced has a tensile strength of at least about 20,000 psi.
9. The method of item 1, wherein at least two active components are added into the continuous mixer.
10. The method of item 1, wherein the encapsulating material is selected from the group consisting of polyvinyl acetate, polyethylene, crosslinked polyvinylpyrrolidone, polymethylmethacrylate, polylactidacid, polyhydroxyalkanoates, ethylcellulose, polyvinyl acetatephthalate, polyethylene glycol esters, methacrylicacid-co-methylmethacrylate and combinations thereof.
11. The method of item 1, wherein the encapsulating material is present in an amount of from about 30% to 99% by weight based on the total weight of the delivery system.
12. The method of item 1, wherein the encapsulating material is present in an amount of from about 60% to 90% by weight based on the total weight of the delivery system.
13. The method of item 1, wherein the at least one active component is selected from the group consisting of a sweetener, an acid, a flavorant, a pharmaceutical, a therapeutic agent, a vitamin, a mineral, a breath freshener, a tooth whitener, a tooth cleaner, a warming agent, a sensate, a cooling agent and combinations thereof.
14. The method of item 13, wherein the at least one active component is a sweetener.
15. The method of item 13, wherein the at least one active component is an acid or a flavor.
16. The method of item 13, wherein the at least one active component is a cooling agent.
17. The method of item 1, which further comprises coating the at least one active component with a coating material which is less miscible with the encapsulating material relative to the miscibility of the least one active component with the encapsulating material.
18. A delivery system manufactured according to the method of item 1.
19. A method of manufacturing an edible composition, comprising mixing the delivery system manufactured of item 18 with at least one edible composition-forming component to produce an edible composition.
20. The method of item 19, wherein the edible composition is selected from the group consisting of a food product, a pharmaceutical composition, a foodstuff, a nutrient- containing composition, a vitamin, a neutraceutical, and a combination thereof.
21. The method of item 20, wherein the edible composition is a confectionery.
22. The method of item 20, wherein the edible composition is a chewing gum.
23. The method of item 19, wherein at least two delivery systems are mixed, with the at least one edible ingredient.
24. The method of item 3, wherein the at least two delivery systems provide a different release rate of the encapsulated active component.
25. An edible composition manufactured according to the method of item 19.
26. A method of manufacturing a delivery system comprising at least one active component encapsulated in an encapsulating material, the method comprising feeding the encapsulating material and the at least one active component into a continuous mixer; wherein the continuous mixer comprises at least a first and a second conveying region and a mixing region between the at least a first and second conveying regions, wherein the first conveying region is upstream from the second conveying region in the continuous mixer, and wherein at least a portion of the encapsulating material is fed into a first conveying region and at least a portion of the at least one active component is fed at or upstream of the mixing region but downstream of where the encapsulating material is fed into the continuous mixer, mixing the encapsulating material and at least one active component in the mixing region; and conveying the encapsulating material and the at least one active component through the second conveying region to produce a delivery system.
27. The method of item 26, wherein the at least one active component is at least partially encapsulated in the encapsulating material.
28. The method of item 26, wherein the at least one active component is completely encapsulated by the encapsulating material.
29. The method of item 26, wherein the temperature of the encapsulating material is maintained at a temperature to minimize degradation of the at least one active component.
30. The method of item 26, further comprising feeding at least a portion of the at least one active component into the continuous mixer at an additional position selected from the group consisting of the first conveying region, the mixing region, the second conveying region, and a combination thereof.
31. The method of item 26, wherein the at least a portion of the at least one active component is fed into the continuous mixer with a side feeder.
32. The method of item 26, which further comprises feeding one or more ingredients selected from the group consisting of a fat, an emulsifier, a plasticizer, a softener, a low molecular weight polymer, a high molecular weight polymer, a wax, and a combination thereof into the first conveying region.
33. The method of item 26, further comprising feeding at least a portion of the at least one active component to the continuous mixer with the encapsulating material.
34. The method of item 26, wherein the first conveying region comprises two or more types of conveying elements.
35. The method of item 34, wherein the conveying elements distal to the mixing region comprise a pitch that is less than the conveying elements proximal to the mixing region.
36. The method of item 26, wherein, the second conveying region comprises two or more types of conveying elements.
37. The method of item 36, wherein the conveying elements proximal to the mixing region comprise a pitch that is greater than the conveying elements distal to the mixing region.
38. The method of item 26, wherein the continuous mixer further comprises a pump after the second conveying region.
39. The method of item 26, wherein the delivery system produced has a tensile strength of at least about 6,500 psi.
40. The method of item 26, wherein the delivery system produced has a tensile strength of at least about 10,000 psi.
41. The method of item 26, wherein at least two active components are fed into the continuous mixer.
42. The method of item 26, wherein the encapsulating material is selected from the group consisting of polyvinyl acetate, polyethylene, crosslinked polyvinyl pyrrolidone, polymethylmethacrylate, polylactidacid, polyhydroxyalkanoates, ethylcellulose, polyvinyl acetatephthalate, polyethylene glycol esters, methacrylicacid-co-methylmethacrylate and combinations thereof.
43. The method of item 26, wherein the encapsulating material is present in an amount of from about 30% to 99% by weight based on the total weight of the delivery system.
44. The method of item 26, wherein the encapsulating material is present in an amount of from about 60% to 90% by weight based on the total weight of the delivery system.
45. The method of item 26, wherein the at least one active component is selected from the group consisting of a sweetener, an acid, a flavorant, a pharmaceutical, a therapeutic agent, a vitamin, a mineral, a breath freshener, a tooth whitener, a tooth cleaner, a warming agent, a sensate, a cooling agent and combinations thereof.
46. The method of item 45, wherein the at least one active component is a sweetener.
47. The method of item 45, wherein the at least one active component is an acid or a flavor.
48. The method of item 45, wherein the at least one active component is a cooling agent.
49. The method of item 26, which further comprises coating the at least one active component with a coating material which is less miscible with the encapsulating material relative to the miscibility of the least one active component with the encapsulating material.
50. A delivery system manufacture according to the method of item 26.
51. A method of manufacturing an edible composition, comprising mixing the delivery system of item 50 with at least one edible composition-forming component to produce an edible composition.
52. The method of item 51, wherein the edible composition is selected from the group consisting of a food product, a pharmaceutical composition, a foodstuff, a nutrient-containing composition, a vitamin, a neutraceutical, and a combination thereof.
53. The method of item 51, wherein the edible composition is a confectionery.
54. The method of item 51, wherein the edible composition is a chewing gum.
55. The method of item 51, wherein at least two delivery systems are mixed with the at least one edible ingredient.
56. The method of item 55, wherein the at least two delivery systems provide a different release rate of the encapsulated active component.
57. An edible composition manufactured according to the method of item 51.

## Claims

1. A method of manufacturing a delivery system comprising at least one active component encapsulated in an encapsulating material, the method comprising:
feeding the encapsulating material and the at least one active component into a continuous mixer in the presence of one or more lubricants; wherein the continuous mixer comprises at least a first and a second conveying region and a mixing region containing one or more mixing elements between the at least a first and second conveying regions, wherein the first conveying region is upstream from the second conveying region in the continuous mixer, and wherein at least a portion of the encapsulating material is fed into a first conveying region and at least a portion of the at least one active component is fed at or upstream of the mixing region but downstream of where the encapsulating material is fed into the continuous mixer,
mixing the encapsulating material and at least one active component in the mixing region; and
conveying the encapsulating material and the at least one active component through the second conveying region to produce a delivery system,
wherein the first conveying region comprises two or more types of conveying elements and wherein the conveying elements in the first conveying region distal to the mixing region comprise a pitch that is less than the conveying elements proximal to the mixing region,
wherein the second conveying region comprises two or more types of conveying elements and wherein the conveying elements in the second conveying region proximal to the mixing region comprise a pitch that is greater than the conveying elements distal to the mixing region.

2. The method of Claim 1, wherein the at least one active component is at least partially encapsulated, or is completely encapsulated, in the encapsulating material.

3. The method of Claim 1, wherein the temperature of the encapsulating material is maintained at a temperature to minimize degradation of the at least one active component.

4. The method of Claim 1, further comprising feeding at least a portion of the at least one active component into the continuous mixer at an additional position selected from the first conveying region, the mixing region, the second conveying region, and a combination thereof.

5. The method of Claim 1, wherein the at least a portion of the at least one active component is fed into the continuous mixer with a side feeder.

6. The method of Claim 1, further comprising feeding at least a portion of the at least one active component to the continuous mixer with the encapsulating material.

7. The method of Claim 1, wherein the continuous mixer further comprises a pump after the second conveying region; and/or wherein the one or more mixing elements are selected from kneading, twin-screw, single-screw, and blade-and-pin.

8. The method of Claim 1, wherein the delivery system produced has a tensile strength of at least 6,500 psi.

9. The method of Claim 1, wherein at least two active components are fed into the continuous mixer.

10. The method of Claim 1, wherein the encapsulating material is selected from polyvinyl acetate, polyethylene, crosslinked polyvinyl pyrrolidone, polymethylmethacrylate, polylactic acid, polyhydroxyalkanoates, ethylcellulose, polyvinyl acetatephthalate, polyethylene glycol esters, methacrylicacid-co-methylmethacrylate and combinations thereof,
and is optionally present in an amount of from about 30% to 99% by weight based on the total weight of the delivery system; and/or
wherein the at least one active component is selected from the group consisting of a sweetener, an acid, a flavorant, a pharmaceutical, a therapeutic agent, a vitamin, a mineral, a breath freshener, a tooth whitener, a tooth cleaner, a warming agent, a sensate, a cooling agent and combinations thereof,
wherein the at least one active component may optionally be coated with a coating material which is less miscible with the encapsulating material relative to the miscibility of the least one active component with the encapsulating material,
wherein the at least one active component is optionally a sweetener.

11. A delivery system manufactured according to the method of Claim 1.

12. A method of manufacturing an edible composition, comprising mixing the delivery system of Claim 11 with at least one edible composition-forming component to produce an edible composition.

13. The method of Claim 12, wherein the edible composition is selected from a food product, a pharmaceutical composition, a foodstuff, a nutrient-containing composition, a vitamin, a nutraceutical, and a combination thereof, or
wherein at least two delivery systems are mixed with the at least one edible ingredient, and wherein the at least two delivery systems optionally provide a different release rate of the encapsulated active component.

14. An edible composition manufactured according to the method of Claim 12.

15. The method of claim 1 wherein the one or more lubricants is selected from glycerol monostearate, hydrogenated vegetable oils and a combination thereof.
